# DEMANDE DE BREVET EUROPEEN

(11) **EP 1 987 860 A2**
(43) Date de publication de la demande: **05.11.2008**
(21) Numéro de dépôt: 08155179.8
(22) Date de dépôt: 25.04.2008
(51) Int. Cl.: A61Q 19/00, A61K 8/73, A61K 8/81, A61K 8/894, A61K 8/99, A23L 1/22, A23L 1/30

(54) **Film hydrosoluble cosmétique**

(30) Priorité: 27.04.2007 FR 0754766
(71) Demandeur: L'ORÉAL, 75008 Paris (FR)
(72) Inventeur: Cassin, Guillaume, 91140, Villebon Sur Yvette (FR); Simonnet, Jean-Thierry, 94230, Cachan (FR)
(74) Mandataire: Le Coupanec, Pascale A.M.P.

(57) **Abrégé**

La présente invention vise une composition se présentant sous la forme d'un film anhydre hydrosoluble comportant (i) au moins un polymère filmogène hydrosoluble ou hydrodispersible et (ii) au moins un microorganisme vivant notamment probiotique. Elle concerne en outre un kit contenant une telle composition en associant avec une composition annexe.

## Description

La présente invention concerne le domaine du soin cosmétique et/ou dermatologique des matières kératiniques et plus particulièrement de la peau.

Plus particulièrement, elle vise à proposer des compositions dédiées au soin des matières kératiniques et mettant en oeuvre des microorganismes.

L'intégration, dans des formules galéniques, de microorganismes tels que les probiotiques, soit vivants, soit sous une forme inactivée, est particulièrement intéressante pour le domaine dermo-cosmétique, en raison des bénéfices que ces microorganismes sont susceptibles d'apporter au niveau de l'organisme hôte.

Ainsi, les documents US 2006/002910, US-2003/049231, WO-A-97/36603, US 6 905 692 et US 6 723 326 proposent de mettre en oeuvre des souches bactériennes spécifiques pour leurs propriétés antifongiques et/ou bactéricides dans des compositions à usage cosmétique ou pharmaceutique. D'une manière générale, les microorganismes sont incorporés dans les compositions correspondantes sous une forme vivante ou inactivée.

Toutefois, l'introduction de microorganismes sous une forme vivante dans une composition soulève des difficultés notamment liées à la nature biologique de ces actifs.

Ainsi, l'introduction de microorganismes vivants dans des compositions galéniques impose que celles-ci soient dépourvues de conservateurs. Or, pour des raisons évidentes, il devient alors très difficile de garantir la propreté microbiologique de la composition correspondante, dans des conditions normales d'utilisation et sur une échelle de temps raisonnable qui peut s'étendre de plusieurs jours à plusieurs semaines.

En outre, on comprend au regard des constatations précédentes, que ces contraintes, liées à la nature biologique des microorganismes, s'opposent à l'utilisation de ceux-ci à des concentrations élevées dans des compositions.

En conséquence, la mise en oeuvre de microorganismes vivants dans des compositions notamment à finalité cosmétique ou dermatologique pose un problème sur le plan de la formulation.

La présente invention vise précisément à proposer une solution à ce problème de galénique.

De manière inattendue, les inventeurs ont observé qu'il est possible de donner satisfaction en ces termes en conditionnant des microorganismes vivants, notamment de type probiotiques, dans une composition cosmétique se présentant sous la forme d'un film anhydre.

A côté des produits cosmétiques habituels se présentant sous forme liquide, gélifiée ou solide, il est déjà connu d'utiliser des préparations cosmétiques sous forme de films fins hydrosolubles, comme décrit par exemple dans le document JP-A-2002/0 212 027, ou dans les documents US 2004/09211, US-A-2002/0127254, US-A-2003/0 175 328 et US-A-2003/0 175 333 qui décrivent l'obtention de films polymériques anhydres pour une administration directe de compositions cosmétiques sur la peau préalablement mouillée. Quant au document US-A-2003/0 186 826, il décrit une composition cosmétique sèche à base de polymères et de tensioactifs, à administrer sur la peau ou les cheveux avec de l'eau. Il est également connu du document FR-A-2 840 221 des films contenant des actifs qui sont libérés en plaçant les films dans un liquide de type eau ou solvant tel que l'huile ou l'éthanol. Par ailleurs, il est connu par le document US-A-2005/0 249 763, des kits comportant un ou plusieurs films fins anhydres et une composition fluide dans laquelle sont introduits ce ou ces films. Au moment de l'application, le ou les films fins anhydres sont dissous extemporanément avec la composition fluide pour former une nouvelle composition à appliquer sur la peau, les muqueuses ou les phanères.

Toutefois, aucun de ces documents n'envisage la mise en oeuvre de tels films pour formuler des microorganismes vivants ni la possibilité d'introduire ceux-ci dans un support galénique en vue de former extemporanément une composition tirant profit des propriétés de ces microorganismes.

Plus précisément, la présente invention a pour objet une composition dite ci-après composition A se présentant sous la forme d'un film anhydre hydrosoluble comportant (i) au moins un polymère filmogène hydrosoluble ou hydrodispersible, et (ii) au moins un microorganisme vivant notamment probiotique.

La présente invention a encore pour objet une composition, dite A, se présentant sous la forme d'un film anhydre hydrosoluble comportant (i) au moins un polymère filmogène hydrosoluble ou hydrodispersible, et (ii) au moins un microorganisme vivant notamment probiotique, et comprenant au moins un dérivé polydiméthylsiloxane oxyalkyléné.

L'invention a aussi pour objet un produit cosmétique obtenu par incorporation d'au moins une composition A telle que définie ci-dessus, dans une seconde composition B comprenant au moins un milieu physiologiquement acceptable.

L'invention a aussi pour objet un kit de formulation d'un produit cosmétique comprenant:
i) au moins une première composition A se présentant sous la forme d'un film anhydre hydrosoluble et telle que définie ci-dessus et
ii) une seconde composition B comprenant un milieu physiologiquement acceptable.

Plus particulièrement, la seconde composition est différente de l'eau pure.

Selon une première variante, le produit est destiné à une administration orale et se présente notamment sous la forme d'un complément alimentaire.

Selon une seconde variante, le produit est destiné à une application topique et se présente sous la forme d'une composition cosmétique et/ou dermatologique selon l'invention.

Ce produit cosmétique peut être formé en ajoutant extemporanément à la composition B, au moins tout ou partie d'un ou plusieurs films (composition A). Les films ajoutés peuvent avoir une composition identique ou différente.

En particulier, le produit cosmétique final est obtenu par mélange extemporané de tout ou partie d'un ou plusieurs films anhydres hydrosolubles formant une composition A et d'une quantité appropriée de la seconde composition B. On entend par « quantité appropriée », une quantité telle que le ou les films s'y dissolvent rapidement. Cette quantité peut aller par exemple de 10 à 1000 mg, de préférence de 50 à 800 mg et mieux de 100 à 500 mg.

Ce mélange peut être effectué directement par l'utilisateur au moment de l'application. Par exemple, l'utilisateur dissout dans sa main ou dans tout autre récipient prévu à cet effet, un ou des film(s) conforme(s) à l'invention en présence d'une quantité appropriée de la composition associée, voire l'intégralité de celle-ci.

La dose appropriée de la seconde composition peut également être obtenue en utilisant des formes de présentation en mono-dose, telles que des sachets, des tubes, des ampoules, des seringues pré-remplies, des capsules molles, des coques ou barquettes en plastique thermoformé. Une dose appropriée peut également être obtenue à partir d'une présentation multi-doses en utilisant un système distribuant une dose pré-définie. Un tel système peut être un flacon pompe, un aérosol, une pipette ou une seringue graduée, un compte-gouttes. Selon cette alternative, la dose peut être distribuée dans la main de l'utilisateur, qui y procède directement à la dissolution du ou des films juste avant administration.

Selon un autre mode de réalisation particulier, le produit est obtenu par incorporation de l'intégralité d'un film formant une composition A conforme à l'invention dans l'intégralité d'une seconde composition B conditionnée par exemple dans un récipient. Le produit ainsi formé peut être stocké pendant plusieurs jours à une température apte à lui garantir une propreté microbiologique par exemple à 4 °C, jusqu'à consommation totale dudit produit.

La présente invention concerne également un procédé de soin cosmétique de matière(s) kératinique(s) comprenant l'administration par voie topique ou orale d'au moins une composition A telle que définie ci-dessus, l'administration étant réalisée dans des conditions propices à la solubilisation de ladite composition, notamment du ou des polymères hydrosolubles ou hydrodispersible formant ladite composition.

Au sens de la présente invention, on entend désigner par l'expression « conditions propices à sa solubilisation », des conditions dans lesquelles le film selon l'invention est associé à une quantité suffisante d'eau ou de milieu aqueux pour conduire sa solubilisation, l'eau ou le milieu aqueux constituant alors la composition B. La solubilisation peut s'effectuer plus particulièrement au contact et dans une composition annexe, notamment cosmétique, contenant un milieu aqueux.

Ainsi, la composition est plus particulièrement administrée sous la forme d'un produit tel que défini précédemment.

Dans un autre mode de réalisation, la solubilisation peut être réalisée par mise en contact de la composition A selon l'invention avec une matière kératinique mouillée ou encore au moment de l'administration par contact avec la salive, l'eau présente sur la matière kératinique ou la salive constituant alors la composition B.

Par « composition cosmétique », on désigne une composition pour application topique, comportant un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice de l'utilisation de cette composition.

Par « milieu physiologiquement acceptable », on désigne un milieu non toxique et susceptible d'être appliqué sur au moins une matière kératinique d'êtres humains.

Par « matières kératiniques », on entend couvrir la peau, les muqueuses, comme les lèvres, les ongles et les fibres kératiniques, à l'image des cils et des cheveux. Les compositions cosmétiques conformes à la présente invention sont particulièrement avantageuses pour une utilisation sur la peau et les lèvres.

Les compositions, produits, kit et procédé selon l'invention s'avèrent particulièrement avantageux dans la mesure où ils sont compatibles avec la mise en oeuvre de microorganismes vivants et permettent de prévenir efficacement sur une durée prolongée dans le temps un risque quelconque de contamination microbiologique ou bactériologique sans requérir à l'usage de conservateurs ou du moins de quantités excessives en conservateurs.

Ils s'avèrent ainsi en outre avantageusement propices à la mise en oeuvre de quantités importantes en microorganismes.

### FILM

Par « film », on entend selon la présente invention, un solide fin. On entend par « fin », un solide ayant une épaisseur d'au maximum 1000 µm.

Ce film est préhensible, c'est-à-dire qu'il a généralement une dimension adéquate pour pouvoir être facilement manipulé par l'utilisateur. Il peut avoir une forme de carré, de rectangle, de disque ou toute autre forme. Un film a généralement une épaisseur de 10 µm à 1000 µm, de préférence de 20 à 500 µm et mieux de 50 à 300 µm Il peut avoir une surface de 0,25 à 25 cm² et de préférence de 2 à 10 cm².

Par ailleurs, par « film anhydre », on entend selon la présente invention, un film contenant moins de 15 % en poids d'eau, de préférence moins de 10 % en poids et mieux moins de 5 % en poids par rapport au poids total du film, et de préférence encore, ne contenant pas d'eau.

En outre, on entend au sens de la présente invention par « film hydrosoluble », un film qui se dissout dans l'eau. Il s'agit d'un film constitué d'un ou de plusieurs polymères hydrosolubles ou hydrodispersibles.

On entend par « hydrosolubles ou hydrodispersibles » des polymères ayant une solubilité dans l'eau mesurée à 25 °C au moins égale à 0,1 gramme/litre (g/L) (obtention d'une solution macroscopiquement isotrope et transparente, colorée ou non). Cette solubilité est de préférence supérieure ou égale à 1 g/L. Les polymères pour constituer ces films peuvent être d'origines synthétique ou naturelle, et être, le cas échéant, modifiés par réactions chimiques. Ils peuvent être ou non filmogènes. Ces polymères doivent être physiologiquement acceptables c'est-à-dire compatible avec la peau, les muqueuses, les cheveux et le cuir chevelu.

Les polymères hydrosolubles utilisés dans le film peuvent être d'origine synthétique ou naturelle, le cas échéant modifiés par réactions chimiques. Ils sont avantageusement filmogènes.

Par « polymère filmogène », on entend un polymère apte à former à lui seul ou en présence d'un agent auxiliaire de filmification, un film continu, et de préférence un film dont la cohésion et les propriétés mécaniques sont telles que ledit film peut être isolé d'un support.

Ces polymères sont catalogués sous la rubrique « Film Formers » dans le dictionnaire cosmétique « International Cosmetic Ingredient Dictionary and Handbook ». (voir par exemple pages 2903 à 2906 de la neuvième édition - 2002).

Les polymères filmogènes peuvent être choisis par exemple parmi :
- les polymères vinyliques tels que l'acétate de polyvinyle, les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle, les copolymères de la vinylpyrrolidone et du caprolactame, les alcools polyvinyliques ;
- les dérivés cellulosiques filmogènes, comme l'hydroxyéthylcellulose, l'hydroxypropylcellulose, l'hydroxypropylméthylcellulose, la méthylcellulose, l'éthylhydroxyéthylcellulose, la carboxyméthylcellulose, et les dérivés quaternisés de la cellulose ;
- les amidons et leurs dérivés;
- les polymères d'origine naturelle, éventuellement modifiés, tels que le pullulane, la pectine, la mannane, les galactomannanes, les glucomannanes et leurs dérivés, la gomme arabique, la gomme de guar, la gomme de xanthane, la gomme de karaya ; les alginates, les carraghénanes, les ulvanes et autres colloïdes algaux ; l'acide hyaluronique et ses dérivés ; la gomme laque, la gomme sandaraque, les dammars, l'élémis, les copals; l'acide désoxyribonucléique ; les mucopolysaccharides tels que l'acide hyaluronique, le sulfate de chondroïtine ;
- les polymères dérivant de la chitine ou du chitosane, anioniques, cationiques, amphotères ou non ioniques,
- les polymères protéiques, tels que les protéines de blé ou de soja; la kératine et ses dérivés, par exemple les hydrolysats de kératine et les kératines sulfoniques; la caséine; l'albumine; le collagène; la gluteline ; le glucagon ; le gluten ; la zéine ; les gélatines et leurs dérivés;
- les copolymères acryliques de phosphoryle choline, tels que le poly-2-(methacryloyloxyethyl) phosphorylcholine commercialisé sous la dénomination Lipidure HM par la société NOF Corporation (nom INCI : Polyphosphorylcholine glycol acrylate) ;
- les complexes anion-cation de type gomme arabique / gélatine ou gomme arabique /chitosane, ou collagène / GlycosAminoGlycane
- et les mélanges de ces polymères.

Selon un mode préféré de réalisation de l'invention, le polymère filmogène est choisi parmi les polymères vinyliques, les dérivés cellulosiques et leurs mélanges.

De manière préférée, le polymère vinylique est l'acétate de polyvinyle (PVA), qui est notamment préparé par polymérisation radicalaire du monomère acétate de vinyle puis hydrolyse. On peut utiliser notamment l'acétate de polyvinyle hydrolysé à 88 %, tel que celui vendu sous la dénomination CELVOL 540 PV ALCOHOL par la société Celanese Chemicals.

De manière préférée, les dérivés cellulosiques sont choisis parmi l'hydroxypropylcellulose (HPC) et l'hydroxypropylméthylcellulose (HPMC). Ces polymères sont solubles dans l'eau ainsi que dans des solvants organiques. Ceci permet d'accroître le champ de solubilité des films les contenant. Le choix du poids moléculaire de ces polymères cellulosiques doit être fait de manière judicieuse pour augmenter la dissolution des films.

Les HPC utilisés de façon préférée sont ceux commercialisées par la société Hercules sous la dénomination :
- Klucel® MF dont le poids moléculaire est 850 000 (viscosité 4000-6500 mPa à 2 % dans l'eau)
- Klucel® EF dont le poids moléculaire est 80 000 (viscosité 300-600 mPa à 10 % dans l'eau)

L'HPMC utilisée de façon préférée est l'hydroxypropylméthylcellulose de viscosité 40-60 cps (40-60 mPa.s) à 2 % dans l'eau à 20 °C, commercialisée par la société Sigma-Aldrich.

De manière préférée, le polymère filmogène est choisi parmi l'acétate de polyvinyle, l'hydroxypropylcellulose, l'hydroxypropylméthylcellulose, et leurs mélanges.

La quantité de polymère(s) filmogène(s) hydrosoluble(s) dans la composition selon l'invention peut aller par exemple de 10 à 95 % en poids, en particulier de 20 à 70 % en poids, et plus particulièrement de 30 à 60 % en poids par rapport au poids total de la composition A.

On peut également utiliser dans la composition de l'invention, un polymère qui soit à la fois un polymère filmogène et un polymère épaississant, choisi par exemple parmi les dérivés cellulosiques et les polymères d'origine naturelle qui peuvent être à la fois filmogènes et épaississants. La quantité d'utilisation reste celle indiquée ci-dessus : par exemple de 10 à 95 % en poids, en particulier de 20 à 70 % en poids, et plus particulièrement de 30 à 60 % en poids par rapport au poids total dudit film.

Selon un mode de réalisation particulier, la composition selon l'invention contient outre le polymère filmogène, au moins un agent épaississant polysaccharidique.

Les agents épaississants polysaccharidiques utilisés dans le film selon l'invention peuvent être choisis parmi les polysaccharides à pouvoir gélifiant.

On définit par « pouvoir gélifiant » le fait qu'à une concentration supérieure ou égale à 0,5 % en poids dans l'eau, la viscosité des solutions ainsi obtenues est supérieure ou égale à 0,01 Pa.s pour un taux de cisaillement égale à 1 s⁻¹, les mesures étant réalisées à 25 °C à l'aide d'un rhéomètre RheoStress RS150 de Haake en configuration cône - plan, les mensurations du cône de mesure étant les suivantes : diamètre : 60 mm et angle : 2°.

Les agents épaississants polysaccharidiques peuvent être choisis notamment parmi la gomme arabique, la gomme de ghatti, la gomme de karaya, la gomme de caroube, la gomme de guar, la gomme de tamarin, la gomme de xanthane, la gellane, les pectines, le tragacanth, l'agar, les alginates, le carrageenan, le furcelleran, le konjac et les dérivés de cellulose, et leurs mélanges.

Selon un mode préféré de réalisation de l'invention, les agents épaississants polysaccharidiques sont choisis parmi les carraghénanes qui sont des polysaccharides linéaires extraits de certaines algues rouges de la famille des Rhodophycée. Ils sont constitués de résidus β-1,3 et α-1,4 galactoses en alternance, de nombreux résidus galactoses pouvant être sulfatés. Il existe trois types de carraghénanes, appelés Kappa-carraghénane, Iota-carraghénane et Lambda-carraghénane. Cette famille de polysaccharides est décrite par exemple dans le chapitre 3 du livre « Food Gels » édité par Peter HARRIS, Elsevier 1989.

On peut en particulier utiliser le carraghénane vendu sous la dénomination SATIAGUM UTC 10 par la société Degussa.

La quantité d'agent(s) épaississant(s) dans la composition A selon l'invention peut aller par exemple de 0,5 à 40 % en poids, en particulier de 1 à 20 % en poids, et plus particulièrement de 5 à 10 % en poids par rapport à son poids total.

En outre, selon un mode de réalisation avantageux, la composition A se présentant sous la forme d'un film peut incorporer en association avec le polymère tel que défini ci-dessus au moins un dérivé polydiméthylsiloxane oxyalkyléné. Comme décrit dans le document (FR0653343) un tel dérivé a pour intérêt d'accroître significativement la cinétique de dissolution du film hydrosoluble l'incorporant.

Les polydiméthylsiloxanes (PDMS) oxyalkylénés utilisés selon l'invention sont hydrosolubles ou hydrodispersibles. On entend par « hydrosolubles ou hydrodispersibles » des PDMS ayant une solubilité dans l'eau mesurée à 25 °C au moins égale à 0,1 gramme/litre (g/1) (obtention d'une solution macroscopiquement isotrope et transparente, colorée ou non). Cette solubilité est de préférence supérieure ou égale à 1 g/1.

Ces PDMS sont choisis de préférence parmi les silicones hydrosolubles comportant au moins un groupement polyoxyalkyléné monovalent terminal ou pendant, et qui, introduits à 0,05 % en poids dans une solution aqueuse, sont susceptibles de réduire la tension superficielle de l'eau à une valeur inférieure à 35 mN/m, et de préférence inférieure à 30 mN/m.

Les PDMS oxyalkylénés convenant à l'invention sont choisis plus préférentiellement parmi les silicones hydrosolubles de formule générale (a) suivante :

R² ₃SiO(R² ₂SiO)ₚ(R²PESiO)_{q}SIR² ₃ (a)

dans laquelle
- les radicaux R2, identiques ou différents, désignent un radical hydrocarboné monovalent choisi parmi les radicaux alkyles, aryles et aralkyles ayant au plus 10 atomes de carbone ; certains des radicaux R2 pouvant également contenir en plus un groupe ethylcyclohexylenemonoxyde de formule 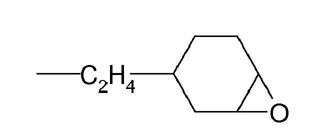 et étant en faible proportion dans la chaîne polysiloxane;
- p varie de 0 à 150, de préférence de 0 à 100 et plus préférentiellement de 0 à 30 ;
- q varie de 1 à 12, de préférence de 1 à 10 et plus préférentiellement de 1 à 8;
- le groupe polyéther PE a la formule (b) suivante

   -CₓH₂ₓ(OC₂H₄)_{y}(OC₃H₆)_{z}OR³ (b)

   dans laquelle :
- x varie de 1 à 8 et de préférence varie de 2 à 4 et plus préférentiellement est égal à 3 ;
- y est supérieur à 0,
- z est supérieur ou égal à 0 ; les valeurs de y et z étant tels que le poids moléculaire total de la portion polyoxyalkylénée du groupe polyéther PE varie de 200 à 10.000 et plus préférentiellement de 350 à 4000 ;
- R3 désigne l'hydrogène, un groupe alkyle en C1-C8 ou un groupe acyle en C2-C8.

Il est à noter que lorsque z est différent de 0, les unités polyoxyéthylène et polyoxypropylène peuvent être distribuées de manière aléatoire le long de la chaîne polyéther PE ou répartis en blocs ou bien à la fois répartis en blocs et de manière aléatoire.

De préférence, les radicaux R² sont choisis parmi les groupes alkyles en C₁-C₄ et les groupes hexyle, phényle et benzyle. Et plus particulièrement, les radicaux R² sont choisis parmi les groupes alkyles et méthyle, éthyle, butyle, encore plus particulièrement ils désignent le radical méthyle.

De préférence, les radicaux R³ sont choisis parmi les groupes alkyles en C₁-C₄ et encore plus particulièrement désignent le radical méthyle.

Les silicones hydrosolubles de formule (a) peuvent être obtenues selon le procédé décrit dans le document US-A-4,847,398.

Parmi les silicones hydrosolubles de formule (a), on utilise de préférence celles de formule (a') suivante :

MeSiO(MeSiO)ₚ(MePESiO)_{q}SIMe₃ (a')

dans laquelle p et q ont les mêmes valeurs qu'indiquées ci-dessus pour la formule (a), et Me désigne le radical méthyle ; PE désigne :

-(CH₂)₃O(OC₂H₄)_{y}(OC₃H₆)_{z}OR³ (b')

où y et z ont les mêmes valeurs qu'indiquées ci-dessus pour la formule b, et R³ désigne hydrogène ou un groupe alkyle en C₁-C₄. et plus particulièrement le radical méthyle.

Comme autre famille de PDMS hydrosolubles utilisables selon l'invention, on peut citer les silicones ramifiées de formule (c) suivante :

(MeSiO)_{q-2}[SiOMe₂)_{p/q} OPE]_{q} (c)

où p et q ont les mêmes valeurs qu'indiquées ci-dessus dans la formule (a) ; Me signifie méthyle ;PE désigne le groupe de formule (d) suivante :

-(OC₂H₄)_{y}(OC₃H₆)_{z}R³ (d)

où y et z ont les mêmes valeurs indiquées ci-dessus dans la formule (b) et R³ désigne un groupe alkyle en C₁-C₄. et plus particulièrement le radical méthyle.

On peut bien sûr utiliser un mélange des silicones de formule (a) et de formule (c).

De tels PDMS oxyalkylénés sont par exemple vendus par la société OSI sous les dénominations commerciales Silwet L-720^{®}, Silwet L-7002^{®}, Silwet L-7600^{®}, Silwet L-7604^{®}, Silwet L-7605^{®}, Silwet L-7607^{®}, Silwet 1614, Silwet L-7657^{®}, Silwet L-7200^{®}, Silwet L7230^{®}, Silsoft 305^{®}, Silsoft 820^{®}, Silsoft 880^{®}, Tegowet 260^{®}, Tegowet 500^{®}, Tegowet 505^{®} et Tegowet 510^{®}.

Le tableau suivant rassemble des valeurs de tensions superficielles à 25 °C de solutions aqueuses comprenant 0,05 % (en poids) de différents PDMS oxyalkylénés :

| PDMS oxyalkyléné | Tension superficielle à 0,05% dans l'eau (mN/m) |
|---|---|
| Tegowet 500^{®} | 33 |
| Tegowet 510^{®} | 29 |
| Silsoft 880^{®} | 26 |
| Silsoft 305^{®} | 21 |

La quantité de polydiméthylsiloxane(s) oxyalkyléné(s) dans la composition A selon l'invention peut aller par exemple de 0,5 à 30 % en poids, de préférence de 1 à 20 % en poids par rapport à son poids total du film.

La composition A selon l'invention peut comprendre en outre un ou plusieurs plastifiants choisis par exemple parmi les polyols tels que la glycérine, le sorbitol, les mono- et/ou di-saccharides, le dipropylène glycol, le butylène glycol, le pentylène glycol, les polyéthylènes glycols tels que le PEG-400. La quantité de plastifiant(s) peut aller par exemple de 1 à 40 % en poids et mieux de 2 à 15 % en poids par rapport à son poids total.

### Microorganismes et notamment microorganismes probiotiques

Les microorganismes convenant à l'invention sont physiologiquement acceptables. En d'autres termes, ce sont des microorganismes qui peuvent être administrés sans risques à l'animal ou l'homme.

En particulier, on utilise dans la présente invention au moins un microorganisme dit de type probiotique.

Au sens de la présente invention, on entend par « microorganisme probiotique », un microorganisme vivant qui, lorsqu'il est consommé en quantité adéquate, a un effet positif sur la santé de son hôte « Joint FAO/WHO Expert Consultation on Evaluation of Health and Nutritional Properties of Probiotic in Food Including Powder Milk with Live Lactic Acid Bacteria, 6 octobre 2001 », et qui peut en particulier améliorer l'équilibre microbien intestinal.

Selon une variante de l'invention, ce microorganisme est mis en oeuvre sous une forme isolée, c'est-à-dire non mélangée à un ou des composé(s) susceptible(s) de lui être associé(s) dans son environnement naturel ou son milieu de culture d'origine.

Les microorganismes convenant à l'invention peuvent être choisis notamment parmi les ascomycètes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus* et leurs mélanges.

Comme ascomycètes convenant tout particulièrement à la présente invention, on peut en particulier citer *Yarrowia lipolitica et Kluyveromyces lactis,* de même que *Saccharomyces cereviseae, Torulaspora, Schizosaccharamyces pombe, Candida* et *Pichia.*

En ce qui concerne les microorganismes probiotiques, ce sont les genres bactériens et de levures suivantes qui sont généralement utilisés :
- les bactéries lactiques, c'est-à-dire qui produisent par fermentation du sucre de l'acide lactique. Suivant leurs morphologies on les divise en deux groupes :
   - *Lactobacillus species : Lactobacillus acidophilus ; amylovorus, casei, rhamnosus, brevis, crispatus, delbrueckii (subsp bulgaricus, lactis), fermentum, helveticus, gallinarum, gasseri johnsonü, paracasei, plantarum, reuteri, salivarius, alimentarius, curvatus, casei subsp. casei, sake,*
   - *Gocci : Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus,*
- Les bifidobactéries ou *Bifidobacterium species comme par exemple les Bifidobacterium adolescentis, animalis, bifidum, breve, lactis, longum, infantis, pseudocatenulatum*
- Les levures *comme par exemple les Saccharomyces (cerevisiae* ou encore *boulardii),*
- Les autres bactéries sporulées *comme par exemple les Bacillus (cereus var toyo ou subtilis), Bacillus coagulans, Bacillus licheniformis, Escherichia coli strain nissle, Propionibacterium freudenreichii,*
- et leurs mélanges.

Les bactéries lactiques et les bifidobactéries sont les probiotiques le plus souvent utilisés.

Des exemples spécifiques de microorganismes probiotiques sont *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus (NCFB 1748) ; Lactobacillus amylovorus, Lactobacillus casei (Shirota), Lactobacillus rhamnosus* (souche GG), *Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonü (CNCM 1-1225), Lactobacillus paracasei, Lactobacillus plantarum, Lactobacillus reuteri, Lactobacillus salivarius, Lactobacillus alimentarius, Lactobacillus curvatus, Lactobacillus casei subsp. casei, Lactobacillus sake Lactococcus lactis, Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus, Saccharomyces (cerevisiae* ou encore *boulardii), Bacillus (cereus var toyo ou subtilis), Bacillus coagulans, Bacillus licheniformis, Escherichia coli strain nissle, Propionibacterium freudenreichii* et leurs mélanges.

Plus particulièrement, il s'agit de microorganismes probiotiques issus du groupe des bactéries lactiques, comme notamment les *Lactobacillus* et/ou les *Bifidobacterium.*

A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges.

Les espèces convenant tout particulièrement sont les *Lactobacillus johnsonii, Lactobacillus paracasei, Bifidobacterium adolescentis, Bifidobacterium longum* et *Bifidobacterum lactis NCC 2818* respectivement déposées suivant le traité de Budapest avec l'Institut Pasteur (28 rue du Docteur Roux, F-75024 Paris cedex 15) les 30/06/92, 12/01/99, 15/04/99, 15/04/99, 07/06/05 sous les désignations suivantes CNCM I-1225, CNCM I-2116, CNCM I-2168 et CNCM I-2170 et CNCM I-3446, et le genre *Bifidobacterium longum (BB536)* et leurs mélanges.

On peut également ajouter les microorganismes de type plancton thermal.

Ce ou ces microorganismes, notamment probiotiques, peuvent être associés à au moins un microorganisme vivant de la flore cutanée.

A titre illustratif de ce type de microorganismes, on peut notamment citer les:
- Staphylococcus epidermis, S. haemolyticus, S. homonis, S. similans,
- Corynobacterium lipophiles, C. jeikeium, C. urealyticum, C. minutissimum,
- Propionobacter granulosum, P. avidum,
- Micrococcus luteus, M. varians
- Streptococcus A, C et G et
- Brevibacterium.

Dans les compositions A selon l'invention, on utilisera généralement 10² à 10¹⁵ ufc/g de microorganisme(s) vivant(s) de la flore cutanée par rapport au poids total de la composition cosmétique sous forme de film.

Selon une autre variante de l'invention, un microorganisme précédent et notamment probiotique peut être associé à au moins une bactérie filamenteuse non photosynthétique ou un de ses extraits.

A titre illustratif de ces bactéries, on peut notamment citer les extraits de bactéries préparés à partir de bactéries filamenteuses non photosynthétiques telles que définies selon la classification du Bergey's Manual of Systematic Bacteriology (Vol. 3, sections 22 et 23, 9ème édition, 1989), parmi lesquelles on peut citer les bactéries appartenant à l'ordre des Beggiatoales, et plus particulièrement les bactéries appartenant aux genres Beggiatoa, Vitreoscilla, Flexithrix ou Leucothrix.

Les bactéries qui viennent d'être définies et dont plusieurs ont déjà été décrites ont généralement un habitat aquatique et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes (ATCC 43181)*
*Beggiatoa alba* (ATCC 33555)
*Flexithrix dorotheae* (ATCC 23163)
*Leucothrix mucor* (ATCC 25107)
*Sphaerotilus natans* (ATCC 13338)

Par 'extrait de bactérie' selon l'invention, on entend un extrait de la biomasse bactérienne ou toute fraction active dudit extrait, en particulier :
(i) des cellules de bactérie isolées du milieu de culture, qui ont été concentrées, par exemple par centrifugation ('extrait cellulaire non stabilisé') ; ou
(ii) des cellules de bactérie concentrées (i), puis soumises à une opération de rupture des enveloppes des cellules de bactérie, par tout moyen connu de l'homme du métier, comme l'action d'ultrasons ou préférentiellement l'autoclavage ('extrait cellulaire stabilisé'). Par 'enveloppes', on entend paroi bactérienne et éventuellement les membranes sous-jacentes ;
(iii) le surnageant obtenu par filtration de l'extrait cellulaire stabilisé (ii), ou toute fraction active dudit extrait.

Ces extraits ou fractions peuvent être conservés par exemple par congélation desdits extraits ou desdites fractions et utilisés après décongélation.

L'extrait de bactérie filamenteuse non photosynthétique utilisable dans la composition utilisée dans le procédé selon l'invention est de préférence choisi parmi un extrait cellulaire, le surnageant dudit extrait cellulaire ou une fraction active dudit extrait cellulaire.

De préférence, l'extrait de bactérie filamenteuse non photosynthétique est un extrait cellulaire de *Vitreoscilla filiformis.*

De préférence, on utilisera un extrait de *Vitreoscillafiliformis* (ATCC 15551).

Pour préparer l'extrait de bactérie selon l'invention, on peut cultiver lesdites bactéries selon les méthodes connues de l'homme du métier, ou se reporter en particulier à la description de la demande de brevet WO-A-94-02158. On obtient un extrait cellulaire dont on peut séparer le surnageant par exemple par filtration et centrifugation. L'extrait peut être utilisé sous forme aqueuse ou sous forme lyophilisée.

Comme précisé précédemment, le ou les microorganisme(s) sont mis en oeuvre selon l'invention sous une forme vivante. Ainsi, au sens de la présente invention, une forme vivante de microorganisme entend couvrir une forme dotée de la capacité à se multiplier sous réserve de la placer dans un environnement propice à la manifestation de cette capacité. Ainsi, au sens de la présente invention, le terme vivant couvre l'état dit « dormance » dans lequel les microorganismes peuvent être placés suite à un traitement physicochimique tel que par exemple leur formulation dans un film anhydre soluble.

Le taux de microorganisme(s) vivant(s), dans la composition sous forme de film anhydre soluble peut être compris entre 10⁻¹ ufc/g et 10¹⁵ ufc/g.

Plus particulièrement, les microorganismes, notamment probiotiques, et/ou leurs fractions et/ou métabolites peuvent être formulés en une quantité équivalente à au moins 10¹ ufc/g, en particulier à des doses variant de 10¹ à 10¹⁵ ufc/g, et plus particulièrement de 10³ à 10¹² ufc/g de la composition les contenant.

Dans le cas où les microorganismes ou leurs extraits, se présentent sous forme d'une suspension aqueuse dont le taux de matière active peut être compris entre 0,1 et 15 %, le taux de cette suspension dans la composition, avant sa mise en forme de film anhydre soluble, pourra être compris entre 1 et 90 %.

Il peut être utile d'ajouter un protecteur de réhydratation, qui va permettre que le microorganisme ne souffre pas pendant la réhydratation du film. Un tel composé a pour effet de protéger les microorganismes de la réhydratation future du film anhydre afin en particulier d'éviter les chocs osmotiques.

A titre d'exemple, on peut citer l'inositol, le mannitol, le glucose, le sucrose, le tréhalose, le maltose, le xylitol, la polyvinylpyrrolidone, l'alcool polyvinylique, la dextrine, la maltodextrine et d'une façon générale, tous les monosaccharides et oligosaccharides (2 à 10 unités).

On peut également citer à cet effet les amidons et les amidons modifiés mais également les glycols : glycérol, sorbitol, adonitol, propylène glycols, dipropylène glycols et butylène glycol, ainsi que les acides aminés et les oligopeptides (2 à 25) tels les glutamates, les aspartates.

On peut également utiliser la cystéine, les ascorbates et les érythorbates ainsi que les cyclodextrines. Ils peuvent être utilisés seuls ou en mélange.

On peut également citer la silice et ses dérivés, les argiles, les dérivés cellulosiques (HEC, HPC, HMPC...), les polymères d'origine naturelle comme les alginates, les xanthanes, la gomme de caroube, les gommes de guar, les pectines, l'agaragar, les carraghénanes ; les polymères d'origine bactérienne comme l'acide hyaluronique, le dextrane, la gellane et les hydrogels tels que les carbomers, les dérivés d'AMPS ...

On peut également utiliser des particules et charges solides telles que :
- certains talcs, tels que le 'Talc K1' de la société NIPPON ou le 'Talc Extra Steamic OOS' de la société LUZENAC;
- certaines séricites, telles que la 'Séricite BC282' de la société WHITTAKER,
- l'hydroxyapatite,
- les microsphères de silice à porosité ouverte ou, de préférence, les microsphères de silice creuses, telles que les 'SILICA BEADS' de la société MAPRECOS,
- les microcapsules de verre ou de céramique 'MACROLITE' de la société 3M.
- les microsphères microporeuses de polymères, qui ont une structure analogue à celle d'une éponge, telles que celles en copolymère d'acrylate réticulé 'Polytrap' de la société DOW CORNING, et celles de polyméthacrylate de méthyle 'MICROPEARL M' ou 'MICROPEARL M 100' de la société SEPPIC;
- les microcapsules de polymères qui comportent une seule cavité fermée et forment un réservoir, qui peut contenir un liquide, notamment un actif cosmétique; elles sont préparées par des procédés connus tels que ceux décrits dans le brevet US-A 3 615 972 et EP-A 0 56219. Elles peuvent être réalisées, par exemple, en polymères ou copolymères d'acides, d'amines ou d'esters monomères à insaturation éthylénique, en polymères urée-formaldéhyde, en polymères ou copolymères de chlorure de vinylidène; à titre d'exemple, on peut citer les microcapsules faites de polymères ou copolymères d'acrylate ou de méthacrylate de méthyle, ou encore de copolymères de chlorure de vinylidène et d'acrylonitrile; parmi ces derniers, on indiquera, notamment, ceux qui contiennent, en poids 20-60 % de motifs dérivés de chlorure de vinylidène, 20-60 % en poids de motifs dérivés d'acrylonitrile et 0-40 % en poids d'autres motifs tels que des motifs dérivés d'un monomère acrylique et/ou styrénique; on peut également utiliser des polymères ou copolymères acryliques réticulés, par exemple dans le cas de polymères comportant un groupement carboxylique, par des diols servant d'agents réticulants; à titre d'exemple, on peut citer les microcapsules en copolymère de chlorure de vinylidène-acrylonitrile 'EXPANCEL' de la société Kemanord Plast, les microcapsules 'Q-MAX' de la société Q-MAX et les microcapsules '3 M' de la société.

Le taux de protecteur(s) de réhydratation, dans la composition à déshydrater, peut varier de 1 à 80 %.

Quant à la forme film anhydre de la composition A, elle peut comprendre de 1 à 70 % en poids notamment de 5 à 60 % en poids, en particulier de 10 à 50 % en poids de protecteur(s) de réhydratation en poids du poids total de ladite forme « film anhydre » à l'état sec.

### Préparation de la composition de type film comprenant au moins un microorganisme

Le film formant la composition A selon l'invention comprenant au moins un microorganisme vivant doit être préparé dans des conditions telles qu'elles ne tuent pas le ou les microorganisme(s).

Par exemple, un tel film peut être préparé en deux étapes, la première étant dédiée à la préparation du mélange devant être transformé à l'état de film et la seconde à, précisément, la formation du film à partir du mélange précédent.

Lors de la première étape, on procède au préalable à la dispersion sous agitation du ou des polymères hydrosolubles ou hydrodispersibles dans de l'eau. L'ensemble est maintenu sous agitation de préférence forte jusqu'à obtention d'un gel homogène. Cette agitation peut par exemple être ajustée à une vitesse de 1 200 à 1 600 rpm avec notamment un mélangeur à turbine, par exemple de type Rayneri.

On procède ensuite à l'introduction du ou des microorganismes considérés et des autres composés si présents tels que par exemple un dérivé polydimethylsiloxane oxyalkyléné tel que le PEG-12 Diméthicone commercialisé sous le nom de Silsoft 880 dont l'avantage est d'améliorer la vitesse de solubilisation du film anhydre, ainsi que par exemple le glycérol, la PVP et le glucose comme protecteurs de réhydratation. L'introduction de chacun des composés est réalisée dans des conditions assurant leur dispersion de manière homogène dans le mélange, en veillant à ne pas déstructurer le gel et en préservant son homogénéité en terme de composition.

Pour la réalisation du film correspondant, on procède au couchage de ce mélange à l'aide d'une étireuse de film, sur un support. L'appareil utilisé peut être un applicateur de film de Braive Instruments. L'épaisseur humide du dépôt, c'est-à-dire à l'application de la composition peut varier de 25 µm à 2000 µm et est généralement de l'ordre de 500 pm.

Le film ainsi obtenu est ensuite placé en étuve pour séchage, la température de séchage étant ajustée pour ne pas tuer les microorganismes vivants.

Selon les variantes de l'invention, les compositions sous forme de film peuvent être administrées par voie topique ou par voie orale.

La mise en oeuvre de ces compositions impose la mise en présence avec une quantité suffisante d'eau ou d'un milieu aqueux pour assurer la solubilisation du ou des polymère(s) hydrosoluble(s) qu'elles contiennent.

Cette eau ou milieu aqueux peuvent se présenter sous différentes formes galéniques, selon le mode d'administration retenu.

Le support galénique peut être de nature diverse selon le type de composition considérée.

Par exemple, dans le cas d'une administration par voie orale, la composition selon l'invention peut être introduite dans tout support alimentaire fluide sous réserve qu'il soit propice à la dissolution du polymère hydrosoluble ou hydrodispersible tel que par exemple le lait, le yaourt, le fromage, les laits fermentés, les boissons, les eaux minérales, les soupes. La composition selon l'invention peut également être destinée aux animaux.

Les compositions selon l'invention peuvent ainsi être formulées avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale dite encore composition B, et notamment pour compléments alimentaires sont connus dans ce domaine et ne font pas ici l'objet d'une description détaillée.

Bien entendu, les compositions B destinées à une administration orale selon l'invention peuvent contenir plusieurs autres actifs.

A titre d'actifs utilisables, on peut citer, les vitamines B3, B5, B6, B8, C, E, ou PP, les caroténoïdes, les curcuminoïdes et la niacine.

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, l'hespéridine, des proanthocyanidines et des anthocyanines.

La composition de type B peut comprendre avantageusement au moins un prébiotique ou un mélange de prébiotiques. Plus particulièrement, ces prébiotiques peuvent être choisis parmi les oligosaccharides, produits à partir du glucose, galactose, xylose, maltose, sucrose, lactose, amidon, xylane, l'hémicellulose, l'inuline, des gommes, de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

Pour leur part, les compositions de type A destinées à une application topique, peuvent être formulées avec une composition annexe de type B pouvant se présenter notamment sous forme de solutions aqueuses, hydroalcooliques ou huileuses, de dispersions du type lotion ou sérum, de suspensions ou émulsions obtenues par dispersion d'une phase grasse dans une phase aqueuse (H/E) ou inversement (E/H), ou de suspensions ou émulsions de consistance liquide ou semi-liquide du type lait, ou de consistance molle, semi-solide ou solide du type crème, de gel aqueux ou encore de microémulsions.

Selon une variante préférée de l'invention, la composition B associée, dite seconde composition, est une composition cosmétique et/ou dermatologique, en d'autres termes une composition apte à procurer un maquillage et/ou un soin au niveau d'une matière kératinique telle que par exemple la peau, les lèvres ou la chevelure. Une telle composition est alors distincte de l'eau pure.

Par exemple une telle composition peut comprendre au moins un composé choisi parmi les agents épaississants, les agents gélifiants, les agents émulsionnants, les matières colorantes et/ou les charges organiques ou inorganiques.

Les compositions notamment cosmétiques et/ou dermatologiques de type B considérées selon l'invention contiennent avantageusement au moins une phase grasse liquide.

Avantageusement, elles peuvent se présenter sous la forme d'émulsion.

Les compositions B selon l'invention peuvent ainsi se présenter par exemple sous forme d'une émulsion obtenue par dispersion d'une phase aqueuse dans une phase grasse (E/H) ou d'une phase grasse dans une phase aqueuse (H/E), de consistance liquide ou semi-liquide du type lait, ou de consistance molle, semi-solide ou solide du type crème ou gel, ou encore d'émulsion multiple (E/H/E ou H/E/H). Ces compositions sont préparées selon les méthodes usuelles.

Selon une variante de réalisation, la seconde composition B se présente plus particulièrement sous la forme d'une émulsion huile-dans-eau, notamment obtenue selon le procédé de température d'inversion de phases selon la technologie PIT.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsionnants sont généralement présents dans la composition, en une proportion pouvant aller par exemple de 0,3 à 30 % en poids, et de préférence de 0,5 à 20 % en poids par rapport au poids total de la composition.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés; les éthers d'alcools gras; les éthers de sucre et d'alcools gras en C₈-C₂₄, et leurs mélanges.

Comme esters de glycéryle et d'acide gras, on peut citer notamment le stéarate de glycéryle (mono-, di- et/ou tri-stéarate de glycéryle) (nom INCI : glyceryl stearate) ou le ricinoléate de glycéryle, et leurs mélanges.

Comme esters de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom INCI : PEG-50 stearate), le monostéarate de polyéthylène glycol 100 OE (nom INCI : PEG-100 stearate) et leurs mélanges.

On peut aussi utiliser des mélanges de ces tensioactifs, comme par exemple le produit contenant du Glyceryl stearate et du PEG-100 stearate, commercialisé sous la dénomination ARLACEL 165 par la société Uniqema, et le produit contenant du Glyceryl stearate (mono-distéarate de glycéryle) et du stéarate de potassium, commercialisé sous la dénomination TEGIN par la société Goldschmidt (nom INCI : glycéryl stéarate SE).

Comme esters d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom INCI : Methyl glucose dioleate) ; l'ester mixte de méthylglucoside et du mélange acide oléique / acide hydroxystéarique (nom INCI : Methyl glucose dioleate/hydroxystearate) ; l'ester de méthylglucoside et d'acide isostéarique (nom INCI : Methyl glucose isostearate) ; l'ester de méthylglucoside et d'acide laurique (nom INCI : Methyl glucose laurate) ; le mélange de monoester et de diester de méthylglucoside et d'acide isostéarique (nom INCI : Methyl glucose sesqui-isostearate) ; le mélange de monoester et de diester de méthylglucoside et d'acide stéarique (nom INCI : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom INCI : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom INCI : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à 22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom INCI Ceteareth-20, Ceteareth-30, et leurs mélanges.

Comme éthers de sucre, on peut citer notamment les alkylpolyglucosides, et par exemple le décylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic et leurs mélanges.

La seconde composition B selon l'invention peut également contenir en tant qu'émulsionnant une quantité avantageuse de polymères amphiphiles.

On entend par polymère amphiphile, tous les polymères comportant à la fois une partie hydrophile et une partie hydrophobe et ayant la propriété de former un film séparant deux liquides de polarité différente et permettant ainsi de stabiliser des dispersions liquide - liquide de type direct, inverse ou multiple. Les polymères amphiphiles convenant plus particulièrement réduisent la tension interfaciale eau/ huile jusqu'à 10 mN/m, et ce quelque soit l'huile. Ces polymères sont ioniques (anioniques ou cationiques) ou amphotères. Ils peuvent être hydrosolubles ou hydrodispersibles. On entend par hydrosoluble le fait qu'ils puissent se disperser dans l'eau sous forme de solution moléculaire. On entend par hydrodispersible le fait qu'ils puissent se disperser dans l'eau sous forme particulaire.

Les polymères amphiphiles conformes à l'invention ont généralement un poids moléculaire moyen en nombre allant de 1 000 à 20 000 000 g/mole, de préférence allant de 20 000 à 8 000 000 et plus préférentiellement encore de 100 000 à 700 000 g/mole. Les quantités de polymères amphiphiles utilisées selon l'invention seront choisies de 0,01 à 20 %, de préférence de 0,1 à 10 % et plus préférentiellement encore de 0,2 % à 5 % en poids, par rapport au poids total de la composition le contenant.

On peut utiliser plus particulièrement les copolymères acrylate/C₁₀-C₃₀--alkylacrylate tels que les produits vendus sous les noms PEMULEN TR1, PEMULEN TR2 et CARBOBOL 1382 par la Société GOODRICH, ou bien leurs mélanges. On pourra utiliser aussi les copolymères acrylate/steareth-20 itaconate et copolymères acrylate/ceteth-20 itaconate vendus sous les noms STRUCTURE 2001 et STRUCTURE 3001 par la Société NATIONAL STARCH. A titre de terpolymèrs pouvant être utilisé, on peut citer le terpolymère acide méthacrylique/acrylate de méthyle/diméthyl m-isopropénylbenzylisocyanate d'alcool béhénylique éthoxylé à 40 OE, (c'est-à-dire comportant 40 groupes oxyéthylénés) vendus par la société AMERCHOL sous les dénominations VISCOPHOBE DB 1000 NP3-NP4.

On peut également citer les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous la dénomination SALCARE SC 80.

Les polymères anioniques utilisables selon l'invention sont par exemple les polymères d'acide isophtalique ou d'acide sulfoisophtalique, et en particulier les copolymères de phtalate/sulfoisophtalate/glycol (par exemple diéthylèneglycol/Phtalate/isophtalate/1,4-cyclohexane-diméthanol) vendus sous les dénominations « Eastman AQ polymer » (AQ35S, AQ38S, AQ55S, AQ48 Ultra) par la société Eastman Chemical.

On peut également citer les polymères amphiphiles comportant au moins un motif acide acryl amido 2-méthylpropane sulfonique (AMPS).

Les polymères d'AMPS amphiphiles selon l'invention sont notamment choisis parmi les polymères amphiphiles d'au moins un monomère acide acrylamidon-méthylpropane sulfonique (AMPS) et d'au moins un co-monomère à insaturation éthylénique comportant au moins une partie hydrophobe ayant de 7 à 30 atomes de carbone, en particulier de 7 à 22 atomes de carbone, voire de 12 à 22 atomes de carbone.

Les polymères d'AMPS amphiphiles selon l'invention ont en général un poids moléculaire en poids allant de 50 000 à 10 000 000 g/mol, en particulier de 100 000 à 8 000 000 g/mol et encore plus particulièrement de 100 000 à 7 000 000 g/mol.

Ils peuvent être réticulés ou non réticulés.

A titre indicatif et sans que cela soit limitatif, on peut citer notamment le copolymère d'AMPS et de méthacrylate d'alcool en C₁₂-C₁₄ éthoxylé (copolymère non réticulé obtenu à partir de Genapol LA-070 et d'AMPS) (nom INCI : Ammonium Acryloyldiméthyltaurate/Laureth-7 méthacrylate copolymer) commercialisé sous la dénomination ARTISTOFLEX LNC par la société Clariant, le copolymère d'AMPS et de méthacrylate de stéaryle éthoxylé (25 EO) (copolymère réticulé par le triméthylolpropane triacrylate, obtenu à partir de Genapol T-250 et d'AMPS) (nom INCI : Ammonium Acryloyldimethyltaurate / Steareth-25 Methacrylate Crosspolymer) commercialisé sous la dénomination ARISTOFLEX HMS par la société Clariant, l'Aristoflex SNC (copolymère AMPS/méthacrylate d'alcool C₁₆/C₁₈ éthoxylés (8 moles EP 80/20 ; nom INCI : Ammonium acryloyldiméthyltaurate/stéareth-8 méthacrylate copolymer) et l'Aristoflex HMB (copolymère AMPS/méthacrylate de béhényl éthoxyle (25 OE), réticulé par le triméthylolpropane triacrylate (TMPTA).

Pour les émulsions E/H, on peut citer par exemple comme émulsionnants, les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination « Dow Corning 5200 Formulation Aid » par la société Dow Corning et le Cetyl dimethicone copolyol vendu sous la dénomination ABIL EM 90R par la société Goldschmidt, ou le mélange polyglycéryl-4 isostéarate/cétyl diméthicone copolyol/hexyllaurate vendu sous la dénomination ABIL WE 09 par la société Goldschmidt. On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 et l'Isolan GPS par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004,. et tel que ceux commercialisés sous les références KSG 210, KSG-310, KSG-320, KSG6330, KSG-340 par la société Shin Etsu. On peut également utiliser des élastomères de silicone polyglycérolés, notamment décrits dans la demande de brevet WO2004/024798, .comme ceux vendus sous les dénominations «KSG-710», «KSG-810», «KSG-820», « KSG-830 », « KSG-840 » par la société Shin Etsu.

Comme émulsifiants adaptés à l'obtention d'une émulsion E/H, conviennent notamment les tensioactifs polyisobutylene à terminaison succinique estérifiée, tels que ceux commercialisés sous les noms de Lubrizol 5603^{®} et Chemcinnate 2000^{®} par les sociétés Lubrizol et Chemron.

Selon un mode de réalisation avantageux, l'émulsion est préparée par la technique d'inversion de phase en température (émulsions PIT). Cette technique permet notamment d'accéder à une taille moyenne des globules constituant la phase huileuse variant de 0,1 à 4 µm (100 à 4000 nm).

L'émulsification par inversion de phase est expliquée en détail dans l'ouvrage T.Förster, W von Rybinski, A.Wadle, Influence of microemulsion phases on the preparation of fine disperse emulsions, Advances in Colloid and interface sciences, 58, 119-149, 1995 cité ici pour référence.

Dans le cas d'une émulsion PIT, le système émulsionnant utilisé dans la seconde composition B selon l'invention comprend un ou plusieurs émulsionnants dont la solubilité dans l'huile augmente avec l'augmentation de la température, ayant un HLB (hydrophilic lipophilic balance) allant de 8 à 18 et de préférence de 10 à 16. Ces émulsionnants sont choisis parmi les alcools gras éthoxylés, les acides gras éthoxylés, les glycérides partiels d'acides gras éthoxylés, les triglycérides d'acides gras polyglycérolés et leurs dérivés éthoxylés, et leurs mélanges.

Les émulsionnants sont de préférence choisis parmi les alcools gras éthoxylés ou les acides gras éthoxylés

Comme alcools gras éthoxylés, on peut citer par exemple les produits d'addition d'oxyde d'éthylène avec l' alcool laurylique, notamment ceux comportant de 9 à 50 groupes oxyéthylénés (Laureth-9 à Laureth-50 en noms INCI) ; les produits d'addition d'oxyde d'éthylène avec l'alcool béhénylique, notamment ceux comportant de 9 à 50 groupes oxyéthylénés (Beheneth-9 à Beheneth-50 en noms INCI); les produits d'addition d'oxyde d'éthylène avec l'alcool cétéarylique (mélange d'alcool cétylique et d'alcool stéarylique), notamment ceux comportant de 9 à 30 groupes oxyéthylénés (Ceteareth-9 à Ceteareth-30 en noms INCI) ; les produits d'addition d'oxyde d'éthylène avec l'alcool cétylique, notamment ceux comportant de 9 à 30 groupes oxyéthylénés (Ceteth-9 à Ceteth-30 en noms INCI) ; les produits d'addition d'oxyde d'éthylène avec l'alcool stéarylique, notamment ceux comportant de 9 à 30 groupes oxyéthylénés (Steareth-9 à Steareth-30 en noms INCI) ; les produits d'addition d'oxyde d'éthylène avec l'alcool isostéarylique, notamment ceux comportant de 9 à 50 groupes oxyéthylénés (Isosteareth-9 à Isosteareth-50 en noms INCI) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms INCI : PEG-9 laurate à PEG-50 laurate) ; les palmitates de PEG-9 à PEG-50 (en noms INCI : PEG-9 palmitate à PEG-50 palmitate) ; les stéarates de PEG-9 à PEG-50 (en noms INCI : PEG-9 stearate à PEG-50 stéarate) ; les palmito-stéarates de PEG-9 à PEG-50 ; les béhénates de PEG-9 à PEG-50 (en noms INCI : PEG-9 behenate à PEG-50 behenate) ; et leurs mélanges.

On peut utiliser aussi des mélanges de ces dérivés oxyéthylénés d'alcools gras et d'acides gras.

De préférence, le système émulsionnant d'une seconde composition de l'invention, se présentant sous la forme d'une émulsion obtenue selon la technique PIT, contient comme émulsionnant au moins un alcool gras éthoxylé, plus particulièrement les Ceteth, ceteareth, beheneth, et leurs mélanges, et plus particulièrement le beheneth-10.

Ce système émulsionnant peut contenir en outre un ou plusieurs co-émulsionnants. Comme co-émulsionnants, on peut citer par exemple les alcools gras ayant 8 à 30 atomes de carbone, comme par exemple l'alcool cétylique, l'alcool stéarylique, l'alcool béhénique ; les acides gras ayant 8 à 30 atomes de carbone, comme par exemple l'acide palmitique, l'acide stéarique, l'acide béhénique ; les esters gras de glycérol, comme par exemple le glycéryl stéarate ; les dérivés oxyéthylénés de ces alcools gras, acides gras et esters gras de glycérol, comportant 2 à 8 groupes oxyde d'éthylène, et leurs mélanges.

Comme exemples d'huiles utilisables dans une composition B selon l'invention, on peut citer :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R₁COOR₂ et R₁OR₂ dans laquelle R₁ représente le reste d'un acide gras comportant de 8 à 29 atomes de carbone, et R₂ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam^{®};
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) .

Les compositions B selon l'invention peuvent comprendre une huile volatile.

Par « huile volatile », on entend au sens de l'invention une huile susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa (10-3 à 300 mm de Hg), en particulier allant de 1,3 Pa à 13 000 Pa (0,01 à 100 mm de Hg), et plus particulièrement allant de 1 ,3 Pa à 1300 Pa (0,01 à 10 mm de Hg).

On peut citer comme huiles volatiles entre autres, les silicones cycliques ou linéaires renfermant de 2 à 6 atomes de silicium, telles que le cyclohexasiloxane, le dodecamethylpentasiloxane, le decamethyltetrasiloxane, le butyltrisiloxane et l'éthyltrisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de « PF 5050^{®} » et « PF 5060^{®} » par la Société 3M et les dérivés de perfluoromorpholine, tels que la 4-trifluorométhyl perfluoromorpholine vendue sous la dénomination « PF 5052^{®} » par la Société 3M.

La quantité de phase huileuse présente dans les compositions B selon l'invention peut aller par exemple de 0,01 à 50 % en poids et de préférence de 0,1 à 30 % en poids par rapport au poids total de la composition.

Les compositions B selon l'invention peuvent en outre comprendre au moins une matière colorante notamment choisie par exemple parmi les pigments, les nacres, les colorants, les matériaux à effet et leurs mélanges.

Ces matières colorantes peuvent être présentes en une teneur allant de 0,01 % à 50 % en poids, de préférence de 0,01 % à 30 % par rapport au poids total de la composition.

Les compositions B selon l'invention peuvent également comprendre une charge organique ou inorganique notamment en une teneur allant de 0,01 % à 50 % en poids, par rapport au poids total de la composition de préférence allant de 0,01 % à 30 % en poids. Ces charges peuvent être minérales ou organiques de toute forme, plaquettaires, sphériques ou oblongues, quelle que soit la forme cristallographique (par exemple feuillet, cubique, hexagonale, orthorombique, ou amorphe). On peut citer la silice, le talc, le mica, le kaolin, la lauroyl-lysine, l'amidon, le nitrure de bore, les poudres de PTFE, les poudres de PMMA, les poudres de résine de methyl silsesquioxane (comme le Tospearl 145A de GE Silicone), les particules hémisphérique creuse de résine de silicone (comme les NLK 500, NLK 506 et NLK 510 de Takemoto Oil and Fat), le sulfate de Baryum, le carbonate de calcium précipité, le carbonate et l'hydro-carbonate de magnésium, l'hydroxyapatite, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les compositions B selon l'invention peuvent en outre contenir divers adjuvants couramment utilisés dans le domaine cosmétique, tels que des séquestrants; des filtres UV ; des parfums; des épaississants, et gélifiants.

Parmi les filtres UV, on peut citer les filtres organiques et/ou minéraux actifs dans l'UVA et/ou l'UVB hydrophiles et/ou lipophiles et/ou bien insolubles dans les solvants cosmétiques couramment utilisés.

Selon la fluidité de la composition B que l'on souhaite obtenir, on peut incorporer dans la composition, un ou plusieurs gélifiants, notamment hydrophiles, c'est-à-dire solubles ou dispersibles dans l'eau.

Comme gélifiants hydrophiles, on peut citer en particulier les polymères épaississants hydrosolubles ou hydrodispersibles. Ceux-ci peuvent notamment être choisis parmi : les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Goodrich ; les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom INCI : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom INCI : Polyacrylamide / C13-14 Isoparaffin / Laureth-7) et sous le nom de SIMULGEL 600 (nom INCI : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose muicrocristalline, la carboxyméthylcellulose, l'hydroxyméthylcellullose et l'hydroxypropylcellulose, et leurs mélanges. On peut aussi utiliser les copolymères d'anhydride maléique tels que le copolymère methyl vinyl ether / anhydride maleique réticulé par 1,9-decadiene (nom INCI : PVM/MA Decadiene Crosspolymer) commercialisé sous la dénomination Stabileze OM^{®} ou 06^{®} par la société ISP. Les polymères amphiphiles mentionnés plus haut peuvent aussi être utilisés comme gélifiants hydrophiles.

Comme gélifiants lipophiles, on peut citer par exemple les argiles modifiées telles que le silicate de magnésium modifié (bentone gel VS38 de RHEOX), l'hectorite modifiée par le chlorure de distéaryl diméthyl ammonium (nom INCI : Disteardimonium hectorite) commercialisé sous la dénomination « bentone 38 CE » par la société RHEOX.

Une composition B selon l'invention peut en outre comprendre un ou des actif(s) cosmétique(s) ou thérapeutique(s) additionnel(s).

On entend par « actif » tout composé ayant un effet bénéfique sur la matière kératinique sur laquelle est appliqué le produit final, notamment sur la peau.

A titre d'exemples et sans que cette liste soit limitative, on peut citer comme actifs ayant une application cosmétique ou dermatologique :
des agents anti-UV, des agents anti-âge/anti-rides (tels que les agents anti-glycation, stimulant la synthèse de macromolécules dermiques ou épidermiques et/ou empêchant leur dégradation, stimulant la prolifération des fibroblastes et/ou des kératinocytes ou stimulant la différenciation des kératinocytes, les myorelaxants), des agents hydratants, des agents desquamants, des agents anti-pollution et anti-radicalaires, des agents anti-transpirants et déodorants, des agents tenseurs, des agents amincissants, des agents agissant sur la microcirculation, les agents agissant sur le métabolisme énergétiques des cellules, les tenseurs, les agents dépigmentants ou pro-pigmentants, les agents desquamants, des agents anti-séborrhéiques, des agents anti-acné ou encore, des agents anti-inflammatoires/anti-irritants.

On peut encore citer tous les actifs connus pour leur activité sur le vieillissement de la peau comme les c-glycosides et dérivés tels que C-β-D-xylopyranoside-2-hydroxy-propane ou le C-α-D-xylopyranoside-2-hydroxy-propane, et en particulier le C-β-B-xylopyranoside-2-hydroxy-propane sous forme de solution à 30 % en matière active dans un mélange eau/propylèneglycol (60/40 % en poids) tel que le produit fabriqué par CHIMEX sous la dénomination commerciale MEXORYL SBB^{®},les agents kératolytiques ou prodésquamants, par exemple les α-hydroxy-acides, les β-hydroxy-acides, les α-céto-acides, les rétinoïdes et leurs esters, le rétinol, l'acide rétinoïque et ses dérivés; les vitamines C, B3 ou PP, B5, E et les dérivés de ces vitamines et notamment leurs esters, la vitamine K et ses dérivés (K1, K2,...), l'adénosine et ses dérivés ; les agents anti-radicaux libres; la DHEA et ses dérivés; le coenzyme Q10; les agents blanchissants et dépigmentants comme l'acide kojique, les dérivés de para-aminophénols, l'arbutine et leurs dérivés, et leurs mélanges.

Il peut également s'agir d'actifs de traitement capillaire tels que par exemple les agents inhibiteurs de la chute du cheveu, les agents stimulateurs de la pousse du cheveu et les agents anti-pelliculaires.

Comme indiqué précédemment, la seconde composition B de l'invention peut se présenter sous toutes les formes galéniques envisageables sous réserve qu'elle soit propice à la dissolution de la composition de type film.

Notamment, une composition B selon l'invention peut avoir la forme d'une solution aqueuse, hydroalcoolique; d'une dispersion du type lotion ou sérum; d'une émulsion eau-dans-huile, huile-dans-eau ou multiple; d'une suspension.

Une seconde composition B selon l'invention se présente avantageusement sous la forme d'une composition de nettoyage, de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, comme des lotion de nettoyage); une composition de maquillage du corps ou du visage telle qu'un fond de teint; une composition pour le bain; une composition désodorisante; une composition après-rasage.

Une composition B selon l'invention peut également se présenter sous la forme d'une composition pour soin capillaire, notamment un shampooing, une lotion traitante, une crème ou un gel coiffant, des lotions restructurantes pour les cheveux, une lotion ou un gel antichute, un shampooing antiparasitaire.

Le mélange des deux compositions A et B respectives selon l'invention peut être appliqué par tout moyen permettant une répartition uniforme et notamment à l'aide d'un coton, d'une tige, d'un pinceau, d'une gaze, d'une spatule ou d'un tampon, , et peut être éliminée par rinçage à l'eau ou à l'aide d'un détergent doux.

Les exemples figurant ci-après sont présents à titre illustratif et non limitatif du domaine de l'invention.

### Exemple 1

### Préparation de compositions A sous forme d'un film conformes à l'invention

La composition de la solution mise en oeuvre pour la formation d'une première composition de films dits Film 1 est la suivante :

| | **Film 1** |
|---|---|
| **Nom** | **Quantité en %** |
| Alcool polyvinylique (Celvol 540 PVA) | 2,0 |
| Glucose | 1,0 |
| Hydroxypropyl cellulose (Klucel EF) | 3,1 |
| Eau | 81,3 |
| Carraghénane | 1,0 |
| Bifidobacterium longum | 3 |
| Amidon de maïs estérifié (Dry Flo Plus) | 1,6 |
| Glycérine | 3,1 |
| Polyéthylène glycol 400 | 1,9 |
| PEG-12 Dimethicone (SILSOFT 880) | 2,0 |

La réalisation du film se fait en deux étapes. Dans un premier temps, on réalise le mélange et dans un second temps, il y a couchage de ce mélange couplé à son séchage pour former le film.

Plus précisément, les deux étapes sont comme suit :
1^{ère} étape : on disperse le(s) polymère(s) dans l'eau et l'ensemble est laissé sous agitation à vitesse et temps nécessaires pour obtenir un gel homogène. Cette étape dure 3 heures avec une agitation forte (1200 à 1600 tpm) à l'aide d'un mélangeur à turbine (Rayneri).
2^{ème} étape: on disperse le PDMS oxyéthyléné, les probiotiques et le carraghénane. Une fois le mélange homogénéisé, on ajoute l'amidon, le PEG 400 et la glycérine en agitant à la même vitesse que précédemment. Cette étape dure 20 minutes.

Le couchage du mélange se fait à l'aide d'une étireuse de film sur une feuille de SILPHAN 5100 M44A sur la face non siliconée. L'appareil utilisé est un applicateur automatique de film de Braive Instruments. L'épaisseur humide choisie est en général 500 µm Après séchage dans un tunnel à circulation d'air chaud à la température d'environ 50 °C, le film obtenu est découpé à la forme désirée, par exemple sous forme de plaquettes carrées de 25 mm de côté.

Après séchage on obtient un film anhydre de 50µm d'épaisseur environ, dont la composition est :

| | **Film 1** |
|---|---|
| **Nom** | **Quantité en %** |
| Alcool polyvinylique (Celvol 540 PVA) | 10,7 |
| Glucose | 5,34 |
| Hydroxypropyl cellulose (Klucel EF) | 16,57 |
| Carraghénane | 5,35 |
| Bifidobacterium longum | 16 |
| Amidon de maïs estérifié (Dry Flo Plus) | 8,55 |
| Glycérine | 16,57 |
| Polyéthylène glycol 400 | 10,2 |
| PEG-12 Dimethicone (SILSOFT 880) | 10,7 |

Trois autres compositions de types film telles que définies ci-après sont également obtenues sur la base du protocole précédent en utilisant à titre de microorganismes les microorganismes précisés ci-après pour chacune d'entre elles.

| | Film 2 | Film 3 | Film 4 | Film 5 |
|---|---|---|---|---|
| **Nom** | **Quantité en %** | **Quantité en %** | **Quantité en %** | **Quantité en %** |
| Alcool polyvinylique (Celvol 540 PVA) | 10,7 | 10,7 | 10,7 | 10,7 |
| Glucose | 5,34 | 5,34 | 5,34 | 5,34 |
| Hydroxypropyl cellulose (Klucel EF) | 16.57 | 16.57 | 16.57 | 16.57 |
| Carraghénane | 5,35 | 5,35 | 5,35 | 5,35 |
| Lactobacillus paracasei | 16 | | | |
| Lactobacillus johnsonii | | 16 | | |
| Bifidobacterium lactis | | | 16 | |
| Saccharomyces cerevisae | | | | 16 |
| Amidon de maïs estérifié (Dry Flo Plus) | 8,55 | 8,55 | 8,55 | 8,55 |
| Glycérine | 16,57 | 16,57 | 16,57 | 16,57 |
| Polyéthylène glycol 400 | 10,2 | 10,2 | 10,2 | 10,2 |
| PEG-12 Dimethicone (SILSOFT 880) | 10,7 | 10,7 | 10,7 | 10,7 |

### Exemple 2 - Produit conforme à l'invention

### - Première composition sous forme de film

- Le film n°1 de l'exemple 1 est mis en oeuvre sous la forme de petit carré de 25 mm de côté.

### - Seconde composition de type sérum hydratant

| Phase A | |
|---|---|
| PVM/MA Decadiene Crosspolymer | |
| (Stabileze de la société ISP) | 0,20 g |
| Gomme de Xanthane | 0,20 g |
| Methyl Paraben | 0,20 g |
| Phenoxy Ethanol | 0,35 g |
| Eau | qsp 100 |
| | |

| Phase B | |
|---|---|
| Triethanolamine | 0,20 g |
| Polyacrylamide et C13-C14 isoparaffine et laureth-7 | |
| (Sepigel 305 de la société Seppic) | 1,00 g |
| Diazolidinyl Urea | 0,30 g |
| Glycérine | 7,00 g |

Mode opératoire de préparation du sérum : La phase A est chauffée à 75°C environ, sous agitation, puis la phase B préalablement préparée est versée dans la phase A. Le chauffage est ensuite stoppé tout en maintenant l'agitation jusqu'au retour à température ambiante. Une légère agitation est alors maintenue pendant 30 minutes.

La composition aqueuse (sérum) et un ou plusieurs films de l'exemple 1 sont présentés sous forme d'un kit.

Lors de l'utilisation, la consommatrice mélange dans le creux de sa main avec les doigts, une ou plusieurs films de l'exemple 1 avec une dose comprise entre 100 et 500 mg de la composition aqueuse pendant une dizaine de secondes.

Elle masse légèrement la surface à traiter de manière à favoriser l'étalement du produit ainsi obtenu, sur la peau.

Le produit final obtenu peut être appliqué sur le visage ou le corps. La peau est alors hydratée.

## Revendications

1. Composition se présentant sous la forme d'un film anhydre hydrosoluble comportant (i) au moins un polymère filmogène hydrosoluble ou hydrodispersible et (ii) au moins un microorganisme vivant notamment probiotique, et comprenant au moins un dérivé polydiméthylsiloxane oxyalkyléné.

2. Composition selon la revendication 1 dont le film possède une épaisseur de 10 µm à 1 000 µm et notamment à 500 µm.

3. Composition selon la revendication 1 ou 2 dans laquelle le polymère filmogène a une solubilité dans l'eau, mesurée à 25 °C au moins égale à 0,1 g/litre.

4. Composition selon l'une quelconque des revendications précédentes dans laquelle le polymère filmogène est choisi parmi les polymères vinyliques, les dérivés cellulosiques, les amidons et leurs dérivés, les polymères d'origine naturelle, éventuellement modifiés, les polymères dérivant de la chitine ou du chitosane, les polymères protéiques, les copolymères acryliques de phosphorylcholine, les complexes anion-cation et leurs mélanges.

5. Composition selon l'une quelconque des revendications précédentes dans laquelle le polymère filmogène est choisi parmi les polymères vinyliques, les dérivés cellulosiques et leurs mélanges, et en particulier est choisi parmi l'acétate de polyvinyle, l'hydroxypropycellulose, l'hydroxypropyméthycellulose et leurs mélanges.

6. Composition selon l'une quelconque des revendications précédentes dans laquelle la quantité en polymère(s) filmogène(s) varie de 10 à 95 % en poids et de préférence de 20 à 70 % en poids par rapport total de la composition.

7. Composition selon l'une quelconque des revendications précédentes dans laquelle ledit microorganisme est choisi parmi les ascomycètes telles *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus* et leurs mélanges.

8. Composition selon l'une quelconque des revendications précédentes, dans laquelle ledit microorganisme est un microorganisme probiotique, en particulier choisi parmi les bactéries lactiques, les bifidobactéries, les levures, les bactéries sporulées et leurs mélanges.

9. Composition selon la revendication précédente, dans laquelle ledit microorganisme probiotique comprend au moins un microorganisme du groupe des *Lactobacillus* ou *Bifidobacterium,* en particulier choisi parmi les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges.

10. Composition selon l'une quelconque des revendications précédentes comprenant de 10⁻¹ ufc/g à 10¹⁵ ufc/g en microorganisme(s) vivant(s).

11. Composition selon l'une quelconque des revendications précédentes comprenant en outre au moins un agent épaississant polysaccharidique.

12. Kit de formulation d'un produit cosmétique comprenant
(i) au moins une première composition dite A telle que définie selon l'une quelconque des revendications 1 à 11 et
(ii) une seconde composition dite B comprenant au moins un milieu physiologiquement acceptable.

13. Kit selon la revendication précédente dans laquelle le produit est destiné à une application topique.

14. Kit selon la revendication 12 ou 13 dans lequel la seconde composition comprend au moins un actif ayant une application cosmétique ou dermatologique.

15. Produit cosmétique obtenu par incorporation d'au moins une composition telle que définie en revendications 1 à 11 dans une seconde composition comprenant au moins un milieu physiologiquement acceptable

16. Procédé de soin cosmétique de matières kératiniques comprenant l'administration par voie topique ou orale d'un produit tel que défini selon la revendication 15.
